(19) Europäisches Patentamt
European Patent Office
Office européen des brevets

(11) Numéro de publication : **0 350 386 B1**

(12) # FASCICULE DE BREVET EUROPEEN

(45) Date de publication du fascicule du brevet : **08.04.92 Bulletin 92/15**

(21) Numéro de dépôt : **89401906.6**

(22) Date de dépôt : **04.07.89**

(51) Int. Cl.$^5$ : **C07C 69/618,** C07C 69/732, C07C 69/734, A61K 7/42, A61K 7/06, A61K 7/00

(54) **Nouveaux dérivés insaturés liposolubles des benzalmalonates et leur utilisation en tant qu'absorbeurs du rayonnement ultra-violet en cosmétique.**

(30) Priorité : **08.07.88 LU 87271**

(43) Date de publication de la demande : **10.01.90 Bulletin 90/02**

(45) Mention de la délivrance du brevet : **08.04.92 Bulletin 92/15**

(84) Etats contractants désignés : **AT BE CH DE FR GB GR IT LI NL SE**

(56) Documents cités :
**EP-A- 0 100 651**
**FR-A- 2 019 952**
**FR-A- 2 515 662**
**GB-A- 1 037 169**
**JOURNAL OF THE CHEMICAL SOCIETY, PERKIN TRANSACTIONS 1, no. 8, août 1985, pages 1627-1635, Chemical Society, Letchworth, GB; S.P. BREUKELMAN et al.: "Preparation and some reactions of 4- and 5-aryl-4,5-dihydropyridazin-3(2H)-ones"**

(73) Titulaire : **L'OREAL**
**14, Rue Royale**
**F-75008 Paris (FR)**

(72) Inventeur : **Forestier, Serge**
**16, Allée Ferdinand Buisson**
**F-77410 Claye-Souilly (FR)**
Inventeur : **Lang, Gérard**
**44, Avenue Lacour 95210**
**Saint-Gratien (FR)**
Inventeur : **Richard, Hervé**
**48, Rue de l'Ermitage**
**F-75020 Paris (FR)**

(74) Mandataire : **Casalonga, Axel et al**
**BUREAU D.A. CASALONGA - JOSSE**
**Morassistrasse 8**
**W-8000 München 5 (DE)**

EP 0 350 386 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention est relative à de nouveaux dérivés insaturés liposolubles des benzalmalonates et à leur utilisation dans le domaine cosmétique en tant qu'absorbeurs du rayonnement ultra-violet pour la protection de l'épiderme humain et des cheveux vis-à-vis du rayonnement solaire, ainsi que pour la stabilisation des compositions cosmétiques contenant des constituants photosensibles.

On sait que les radiations lumineuses de longueurs d'onde comprises entre 280 nm et 400 nm permettent le brunissement de l'épiderme humain et que les rayons de longueurs d'onde comprises entre 280 et 320 nm, connues sous la dénomination d'UV-B, provoquent des érythèmes et des brûlures cutanées qui peuvent nuire au développement du bronzage; ce rayonnement UV-B doit donc être filtré.

On sait également que les rayons UV-A, de longueurs d'onde comprises entre 320 et 400 nm, provoquant le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photoallergiques.

Il est donc intéressant de disposer de composés absorbant les rayons UV sur une large bande afin de pouvoir filtrer à la fois les rayons UV-A et UV-B.

On sait par ailleurs que les constituants entrant dans les préparations cosmétiques ne possèdent pas toujours une stabilité suffisante à la lumière et qu'ils se dégradent sous l'action des radiations lumineuses.

Par conséquent, il est souhaitable d'incorporer à ces préparations des composés susceptibles de filtrer les rayons UV et qui doivent présenter en outre une bonne stabilité et une solubilité suffisante dans les milieux habituellement utilisés en cosmétique, et en particulier dans les huiles et graisses.

Il est également souhaitable d'assurer aux cheveux une bonne protection contre la dégradation photochimique afin d'éviter en particulier une décoloration ou un changement de nuance.

C'est ainsi que la demanderesse a découvert de manière surprenante, au cours de ses recherches, que certains dérivés liposolubles insaturés des benzalmalonates présentaient de bonnes propriétés filtrantes dans une large gamme de longueurs d'onde allant de 280 à 360 nm. Outre leurs bonnes propriétés filtrantes, les nouveaux dérivés liposolubles de benzalmalonates présentent une excellente stabilité chimique et photochimique et ont l'avantage de n'être ni toxiques, ni irritants et d'avoir une parfaite innocuité vis-à-vis de la peau.

Ils présentent également un excellent caractère liposoluble, ce qui les rend utilisables dans les supports gras utilisés en cosmétique et en particulier dans les compositions destinées à protéger l'épiderme humain contre les rayons UV, et plus particulièrement dans les compositions anti-solaires.

La présente invention a donc pour objet les nouveaux dérivés insaturés liposolubles des benzalmalonates de formule :

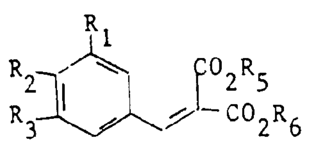

(I)

dans laquelle :

$R_1$ et $R_2$ représentent un atome d'hydrogène, un radical hydroxyle, un radical triméthylsiloxy, un radical alkyle en $C_1$-$C_6$ à chaîne droite ou ramifiée, un radical alcoxy en $C_1$-$C_6$ à chaîne droite ou ramifiée ou un radical $-(CH_2)_p-C(R_4)=CH_2$ dans lequel p représente un nombre entier compris entre et 1 et 10 et de préférence entre 1 et 4 et $R_4$ représente un atome d'hydrogène ou un radical alkyle en $C_1$-$C_4$ à chaîne droite ou ramifiée, l'un des deux radicaux $R_1$ ou $R_2$ représentant un reste $-(CH_2)_p-C(R_4)=CH_2$,

$R_3$ représente un atome d'hydrogène, un radical alkyle en $C_1$-$C_6$ à chaîne droite ou ramifiée ou un radical alcoxy en $C_1$-$C_6$ à chaîne droite ou ramifiée,

$R_5$ et $R_5$ identiques ou différents, représentent un radical alkyle en $C_1$-$C_8$ à chaîne droite ou ramifiée.

Parmi les radicaux alcoxy en $C_1$- $C_6$ à chaîne droite ou ramifiée, on peut citer par exemple les radicaux méthoxy, éthoxy, n-propoxy, isopropoxy, n-butoxy, isobutoxy, tert.-butoxy, n-amyloxy, isoamyloxy, néopentyloxy et n-hexyloxy.

Parmi les radicaux alkyle en $C_1$-$C_6$ à chaîne droite ou ramifiée, on peut citer plus particulièrement les radicaux méthyle, éthyle, n-propyle, isopropyle, n-butyle, isobutyle, tert.-butyle, n-amyle, isoamyle, néopentyle et n-hexyle, et parmi les radicaux alkyle en $C_1$-$C_8$, les radicaux précédents ainsi que les radicaux n-heptyle, n-octyle et 2-éthylhexyle.

Parmi les composés préférés de l'invention de formule (I), on peut citer les composés suivants :

- le 3-allyl-4-hydroxy-benzalmalonate de diéthyle,
- le 3-allyl-4-méthoxy-benzalmalonate de diéthyle,
- le 3-méthallyl-4-méthoxy-benzalmalonate de diéthyle,
- le-4-allyl-benzalmalonate de diéthyle,
- le 4-méthallyl-benzalmalonate de diéthyle,
- le 3-allyl-4,5-diméthoxy-benzalmalonate de diéthyle,
- le 3-allyl-4-butoxy-5-méthoxy-benzalmalonate de diéthyle,
- le 3-allyl-4,5-diméthoxy-benzalmalonate de di-(2-éthylhexyle).

Les composés de formule (I) selon l'invention sont préparés par réaction de KNOEVENAGEL, à savoir condensation d'un aldéhyde aromatique (II) avec un diester d' acide malonique de formule (III) dans le toluène en présence d'acétate de pipéridinium comme catalyseur. L'eau est éliminée par azéotropie. Le schéma réactionnel est le suivant :

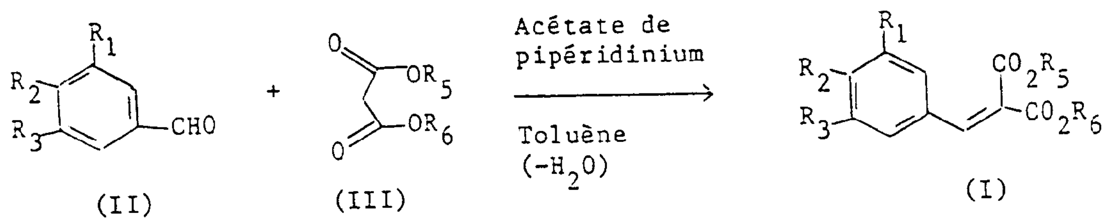

(II) (III) (I)

$R_1$, $R_2$, $R_3$, $R_5$ et $R_6$ ayant les significations indiquées ci-dessus pour la formule (I).

Les produits sont recristallisés, distillés ou séparés par chromatographie sur colonne. Les aldéhydes de formule (II), qui sont des composés connus, peuvent être obtenus selon l'une des méthodes suivantes :

Première méthode

L'aldéhyde de formule (II) dans laquelle $R_1$ représente un reste $-(CH_2)_p-C(R_4)=CH_2$ lorsque p = 1, $R_2$ représente un reste hydroxyle et $R_3$ a la signification mentionnée ci-dessus, ayant la formule (IIA), peut être obtenu par réarrangement de CLAISEN d'un aldéhyde de formule (IV) selon le schéma réactionnel suivant :

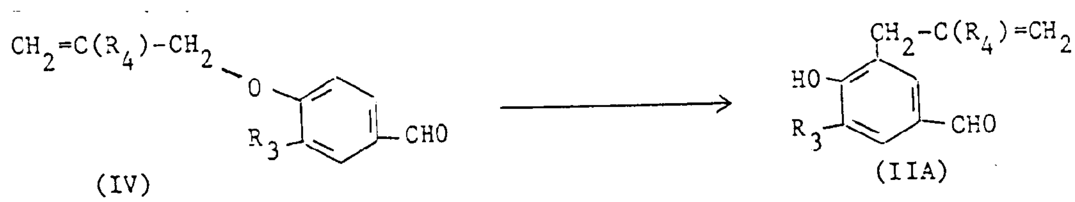
(IV) (IIA)

Ce réarrangement peut être effectué dans les conditions décrites par TARBELL (organic Reactions, vol. 2, John Wiley, New York, 1944, page 1) par chauffage à au moins 170°C environ du composé de formule (IV), éventuellement en présence d'un solvant.

L'aldéhyde de formule (IV) peut être obtenu par réaction d'un halogénure d'alcényle de formule (V) sur un aldéhyde de formule (VI) :

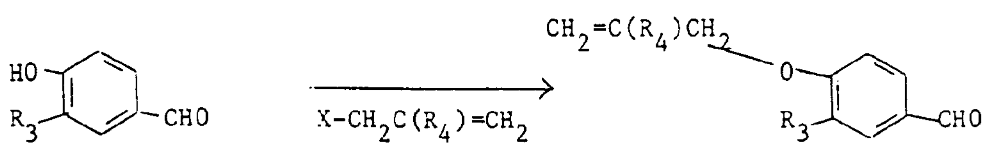

(VI) (V) (IV)

Cette réaction est effectuée en présence d'une base dans un solvant, par exemple en présence d'un carbonate de métal alcalin dans le diméthylformamide, à une température comprise entre la température ambiante et le point d'ébullition du solvant. L'aldéhyde de formule (VI) peut être préparé selon des méthodes connues. Dans le composé de formule (V), X représente un atome d'halogène, de préférence un atome de chlore ou de brome.

Deuxième méthode

L'aldéhyde de formule (IIB) répondant à la formule II dans laquelle $R_1$ représente un reste $-(CH_2)_p-C(R_4)=CH_2$ lorsque p = 1, $R_2$ représente un reste alcoxy en $C_1$-$C_6$ et $R_3$ a la signification mentionnée ci-dessus, peut être obtenu selon l'une des deux voies ci-dessous :

Première voie

Par formylation d'un éther de phénol de formule (VII) selon le schéma réactionnel suivant :

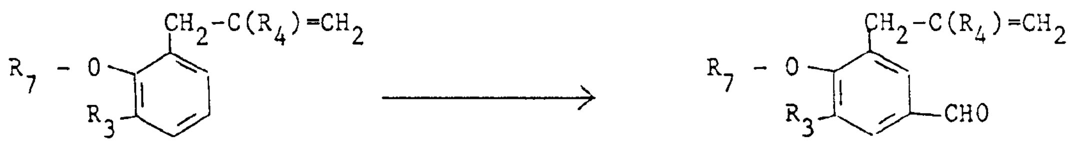

(VII) (IIB)

$R_7$ représente un radical alkyle en $C_1$-$C_6$, $R_3$ ayant la signification mentionnée ci-dessus.

Cette réaction est effectuée par exemple grâce à l'addition des complexes, formés par l'action de l'oxychlorure de phosphore sur les formamides disubstitués selon VILSMEIER et HAACK (Ber., 60, p 119, 1927), sur les composés de formule (VII).

L'éther de phénol (VII) peut être préparé selon des méthodes connues.

Deuxième voie

Le composé de formule ($II_A$) obtenu par la première méthode peut être transformé en composé de formule (IIB) par réaction avec un halogénure ou un sulfate d'alkyle en $C_1$-$C_6$ en présence d'une base, par exemple en présence d'un carbonate de métal alcalin, dans un solvant tel que le diméthylformamide, ou bien en présence d'un hydrure de métal alcalin dans le 1,2-diméthoxyéthane, selon le schéma réactionnel suivant :

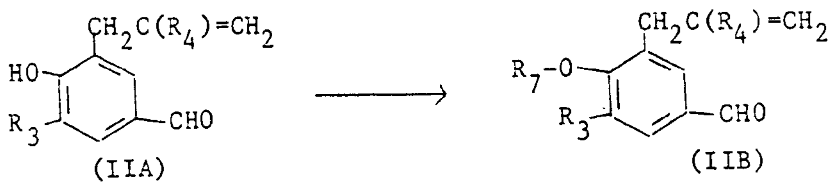

(IIA) (IIB)

Troisième méthode

L'aldéhyde de formule (II) dans laquelle $R_1$ ou $R_2$ représente un reste $-(CH_2)_p-C(R_4)=CH_2$ et $R_3$ représente un atome d hydrogène, un reste alkyle en $C_1$-$C_6$ ou un reste alcoxy en $C_1$-$C_6$ peut également être obtenu par réaction de l'orthoformiate d'éthyle sur un bromure de phénylmagnésium de formule (VIII), suivie d'une hydrolyse de l'acétal formé :

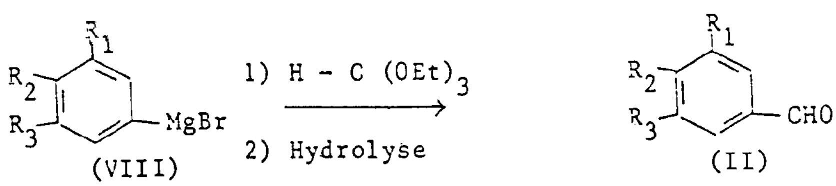


(VIII) (II)

Cette réaction peut être effectuée dans les conditions décrites par QUELET (C.R.Acad.Sci. vol. 182, p 1285 et Bull.Soc.Chim.Fr. vol .45 p 267), par exemple dans un solvant inerte tel que l'éther éthylique, le dioxane ou le 1,2-diméthoxyéthane, à une température comprise entre la température ambiante et le point d'ébullition du solvant. Dans les composés de formule (II) et (VIII), l'un des substituants $R_1$ ou $R_2$ représente un radical $-(CH_2)_p-C(R_4)=CH_2$, $R_4$ et p ayant les significations mentionnées ci-dessus, et l'autre représente un atome d'hydrogène, un radical alkyle en $C_1$-$C_6$ ou un radical alcoxy en $C_1$-$C_6$ et $R_3$ représente un atome d'hydrogène, un radical

alkyle en $C_1$-$C_6$ ou un radical alcoxy en $C_1$-$C_6$.

De par leur caractère liposoluble, les dérivés insaturés de benzalmalonates de formule (I) ci-dessus se répartissent uniformément dans les supports cosmétiques classiques contenant au moins une phase grasse et peuvent être appliqués sur la peau ou les cheveux pour constituer un film protecteur efficace.

La présente invention a donc aussi pour objet une composition cosmétique comprenant, dans un support cosmétiquement acceptable contenant au moins une phase grasse, une quantité efficace d'au moins un dérivé insaturé de benzalmalonate de formule (I) ci-dessus.

La composition cosmétique de l'invention peut être utilisée comme composition protectrice de l'épiderme humain ou des cheveux ou comme composition anti-solaire.

La présente invention a également pour objet un procédé de protection de la peau et des cheveux naturels ou sensibilisés vis-à-vis du rayonnement solaire, consistant à appliquer sur la peau ou les cheveux une quantité efficace d'au moins un composé de formule (I) contenu dans un support cosmétiquement acceptable contenant au moins une phase grasse.

On entend par "cheveux sensibilisés" des cheveux ayant subi un traitement de permanente, de coloration ou de décoloration.

L'invention a également pour objet une composition cosmétique colorée ou non colorée, stabilisée à la lumière, comprenant une quantité efficace d'au moins un dérivé de benzalmalonate de formule (I) ci-dessus.

Lorsqu'elle est utilisée comme composition destinée à protéger l'épiderme humain contre les rayons ultraviolets, la composition cosmétique selon l'invention peut se présenter sous les formes les plus diverses habituellement utilisées pour ce type de composition. Elle peut notamment se présenter sous forme de lotions huileuses ou oléoalcooliques, d'émulsions telles qu'une crème ou un lait, de gels oléoalcooliques, alcooliques ou hydroalcooliques, de bâtonnets solides, ou être conditionnée en aérosol.

Elle peut contenir les adjuvants cosmétiques habituellement utilisés dans ce type de composition tels que des épaississants, des adoucissants, des humectants, des tensio-actifs, des conservateurs, des anti-mousses, des parfums, des huiles, des cires, de la lanoline, des propulseurs, des colorants et/ou pigments ayant pour fonction de colorer la composition elle-même ou la peau ou tout autre ingrédient habituellement utilisé en cosmétique.

Le composé de formule (I) est présent dans des proportions en poids comprises entre 0,25 et 3% par rapport au poids total de la composition cosmétique protectrice de l'épiderme humain.

Comme solvant de solubilisation, on peut utiliser une huile, une cire et de façon générale tout corps gras, un monoalcool ou un polyol inférieur ou leurs mélanges. Les monoalcools ou polyols plus particulièrement préférés sont l'éthanol, l'isopropanol, le propylèneglycol, la glycérine et le sorbitol.

Une forme de réalisation de l'invention est une émulsion sous forme de crème ou de lait protecteurs comprenant en plus du composé de formule (I), des alcools gras, des esters d'acides gras et notamment des triglycérides d'acides gras, des acides gras, de la lanoline, des huiles ou cires naturelles ou synthétiques et des émulsionnants, en présence d'eau.

Une autre forme de réalisation est constituée par des lotions huileuses à base d'huiles et cires naturelles ou synthétiques, de lanoline, et d'esters d'acides gras, notamment de triglycérides d'acides gras, ou par des lotions oléoalcooliques à base d'un alcool inférieur tel que l'éthanol ou d'un glycol tel que le propylèneglycol et/ou d'un polyol tel que la glycérine et d'huiles, de cires et d'esters d'acides gras tels que les triglycérides d'acides gras.

La composition cosmétique de l'invention peut également être un gel alcoolique comprenant un ou plusieurs alcools ou polyols inférieurs tels que l'éthanol, le propylèneglycol ou la glycérine et un épaississant tel que la silice. Les gels oléoalcooliques contiennent en outre une huile ou une cire naturelle ou synthétique.

Les bâtonnets solides sont constitués de cires et d'huiles naturelles synthétiques, d'alcools gras, d'esters d'acides gras, de lanoline et autres corps gras. Dans le cas d'une composition conditionnée en aérosol, on utilise les propulseurs classiques tels que les alcanes, les fluoroalcanes et les chlorofluoroalcanes.

La présente invention vise également les compositions cosmétiques anti-solaires contenant au moins un composé de formule (I) et pouvant contenir d'autres filtres UV-B et/ou UV-A.

Dans ce cas, la quantité totale de filtres présents dans la composition anti-solaire, c'est-à-dire le composé de formule (I) et éventuellement les autres filtres, est comprise entre 0,5 et 15% en poids par rapport au poids total de la composition anti-solaire.

Ces compositions anti-solaires se présentent sous les formes indiquées ci-dessus pour les compositions protectrices de l'épiderme humain.

Lorsque la composition cosmétique selon l'invention est destinée à protéger des rayons UV les cheveux naturels ou sensibilisés, cette composition peut se présenter sous forme de shampooing, de lotion, gel ou émulsion à rincer, à appliquer avant ou après le shampooing, avant ou après coloration ou décoloration, avant ou après permanente, de lotion ou gel coiffants ou traitants, de lotion ou gel pour le brushing ou la mise en plis,

de laque pour cheveux, de composition de permanente, de coloration ou décoloration des cheveux. Cette composition peut contenir, outre le composé de l'invention, divers adjuvants utilisés dans ce type de composition, tels que des agents tensio-actifs, des épaississants, des polymères, des adoucissants, des conservateurs, des stabilisateurs de mousse, des électrolytes, des solvants organiques, des dérivés siliconés, des huiles, des cires, des agents anti-gras, des colorants et/ou pigments ayant pour fonction de colorer la composition elle-même ou la chevelure ou tout autre ingrédient habituellement utilisé dans le domaine capillaire.

Elle contient 0,25 à 3% en poids de composé de formule (I).

La présente invention vise également les compositions cosmétiques contenant au moins un composé de formule (I) à titre d'agent de protection contre les rayons ultraviolets constituées par des compositions capillaires telles que les laques pour cheveux, les lotions de mise en plis éventuellement traitantes ou démêlantes, les shampooings colorants, les compositions tinctoriales pour cheveux; par des produits de maquillage tels que les vernis à ongles, les crèmes et huiles de traitement pour l'épiderme, les fonds de teint, les bâtons de rouge à lèvre, les compositions pour les soins de la peau telles que des huiles ou crèmes pour le bain, ainsi que toute autre composition cosmétique pouvant présenter du fait de ses constituants, des problèmes de stabilité à la lumière au cours du stockage.

De telles compositions contiennent 0,25 à 3% en poids de composé de formule (I).

L'invention vise également un procédé de protection des compositions cosmétiques contre les rayons ultraviolets, consistant à incorporer à ces compositions une quantité efficace d'au moins un composé de formule (I).

L'invention sera mieux illustrée, sans toutefois être limitée, par les exemples de réalisation suivants.

EXEMPLES DE PREPARATION

EXEMPLE 1 :

3-allyl-4-méthoxy-benzalmalonate de diéthyle

Préparation d'un composé de formule générale (I) dans laquelle $R_1$ représente le radical $-CH_2-CH=CH_2$, $R_2$ représente le radical $-OCH_3$, $R_3$ représente un atome d'hydrogène et $R_5$ et $R_5$ représentent le radical-$C_2H_5$ :

Premier stade :

Préparation du 3-allyl-4-méthoxy-benzaldéhyde

Première méthode

On chauffe pendant 4 heures sous azote et sous agitation à 220°C, 50 g (0,308 mole) de 4-allyloxybenzaldéhyde. Le mélange réactionnel refroidi est repris dans du dichlorométhane et extrait à la soude 5N. La phase aqueuse est acidifiée à l'acide chlorhydrique 6N et extraite au dichlorométhane. La phase organique est séchée et le solvant évaporé pour donner une huile brun noir. Après distillation sous vide, on recueille la fraction Eb = 138-140°C sous 106 Pa (15g, Rendement = 30%) de 3-allyl-4-hydroxy-benzaldéhyde (Poudre blanche, Pf = 66°C).

On introduit successivement le dérivé précédent (14,5 g, 0,089 mole), 30 ml de N,N-diméthylformamide, 13,6 g (0,098 mole) de carbonate de potassium anhydre et 11 ml (0,178 mole) d'iodure de méthyle. On porte le tout à 60-70°C pendant 3 heures. On verse le mélange réactionnel dans de l'eau glacée et on extrait le tout à l'éther diisopropylique. On sèche la phase organique sur sulfate de sodium, on filtre et évapore le solvant pour obtenir le 3-allyl-4-méthoxybenzaldéhyde (huile jaune pâle, 13,6 g, rendement = 87%).

Deuxième méthode :

Dans un réacteur de 5 litres, on introduit successivement du 2-allylphénol (100 g, 0,75 mole), 2 litres de N,N-diméthylformamide sec et du carbonate de potassium anhydre (206 g, 1,49 mole). A température ambiante, on introduit goutte à goutte de l'iodure de méthyle (92 ml, 1,49 mole). On laisse pendant 4 heures à 38°C. On verse le mélange réactionnel dans de l'eau glacée et on extrait au dichlorométhane. La phase organique est lavée à l'eau et séchée. Après évaporation du solvant et distillation sous vide, on récupère une fraction distillant à 110°C sous 5000 Pa de 2-allylanisole (liquide incolore, 46 g, rendement = 42%).

Dans un réacteur de 500 ml, on place du N,N-diméthylformamide (75 ml, 0,98 mole) et on ajoute, tout en

refroidissant vers 5°C, de l'oxychlorure de phosphore (26 ml, 0,28 mole). On maintient le mélange une heure à 10°C et on introduit goutte à goutte le dérivé précédent (41,5 g, 0,28 mole). On monte progressivement la température à 100°C en une heure et on maintient le mélange réactionnel à cette température pendant 10 heures. Le mélange refroidi est versé dans de l'eau glacée et extrait à l'éther diisopropylique. Les phases organiques sont lavées à l'eau, séchées sur sulfate de sodium, filtrées et le solvant évaporé pour donner un produit brut (31 g), lequel est purifié par chromatographie sur silice 60 (éluant : toluène/hexane 50:50) pour donner une fraction (4,5 g) de 3-allyl-4-méthoxy-benzaldéhyde identique à celui obtenu par la première méthode.

Deuxième stade :

Préparation du 3-allyl-4-méthoxy-benzalmalonate de diéthyle

On chauffe au reflux, sous azote avec un Dean Stark, un mélange du dérivé précédent (10g, 0,057 mole), de malonate de diéthyle (9,09 g, 0,057 mole), de toluène (15 ml), d'acide acétique (0,36 ml) et de pipéridine (0,68 ml). Après 5 heures de chauffage, on a recueilli 1 ml d'eau. Après refroidissement, on lave la phase toluénique à l'eau, on la sèche et on distille le solvant. On obtient une huile orangée qui cristallise. On la recristallise dans l'éther diisopropylique avec traitement au noir animal. On obtient des cristaux blancs de 3-allyl-4-méthoxy benzalmalonate de diéthyle (12,7 g, rendement = 70%) possédant les caractéristiques suivantes :

- Point de fusion 69°C
- Spectre de RMN 1H ($CDCl_3$) : spectre conforme à la structure attendue.

- Spectre UV ($CHCl_3$) : $\lambda_{max}$ = 318 nm
  $\varepsilon$ = 24450

- Analyse élémentaire :

calculé : C 67,91; H 6,97; 0 25,13
trouvé : C 68,04; H 6,89; 0 25,23.

EXEMPLE 2 :

3-allyl-4,5-diméthoxy-benzalmalonate de diéthyle

Préparation d'un composé de formule générale (I) dans laquelle $R_1$ représente le radical $-CH_2-CH=CH_2$, $R_2$ et $R_3$ représentent le radical $-OCH_3$ et $R_5$ et $R_6$ représentent le radical-$C_2H_5$

Premier stade :

On porte à 180°C pendant 6 heures 30 minutes sous agitation le 4-allyloxy-3-méthoxy-benzaldéhyde (62,5 g, 0,325 mole). On refroidit. Le solide marron est repris dans le dichlorométhane et extrait à la soude à 5%. La phase aqueuse est acidifiée à l'acide chlorhydrique 3N. Le solide obtenu est filtré et recristallisé dans un mélange éthanol/eau 40:60. On obtient le 3-allyl-4-hydroxy-5-méthoxy-benzaldéhyde (poudre beige clair, 62,5 g, rendement = 71%, point de fusion = 83-84°C).

Deuxième stade

On introduit successivement dans un réacteur le dérivé précédent (34 g, 0,18 mole), le diméthylformamide (500 ml), le carbonate de potassium (49 g, 0,35 mole) et l'iodure de méthyle (50 g, 0,35 mole). On maintient à une température de 40°C pendant 3 heures. On plonge le mélange réactionnel dans de l'eau glacée et on extrait l'huile formée par du dichlorométhane. Après lavage, séchage et évaporation du solvant, on obtient une huile brun clair, laquelle est passée sur lit de silice 60 pour donner une huile jaune pâle de 3-allyl-4,5-diméthoxy-benzaldéhyde (34,3 g, rendement = 92%).

Troisième stade :

On chauffe pendant 7 heures au reflux avec un Dean Stark un mélange du dérivé précédent (15 g 0,073

mole), de malonate de diéthyle (11,7 g, 0,073 mole), de toluène (18 ml), d'acide acétique (0,46 ml) et de pipéridine (0,87 ml). Après refroidissement, on lave la phase toluénique à l'eau, on la sèche et on distille le solvant. L'huile orange pâle obtenue (24,5 g, rendement = 96%) est cristallisée dans un mélange éther diisopropylique/hexane 50:50 pour donner des cristaux blancs de 3-allyl-4,5-diméthoxy-benzalmalonate de diéthyle (14,2 g, rendement = 56%) possédant les caractéristiques suivantes :

- Point de fusion : 43-44°C
- Spectre de RMN 1H ($CDCl_3$) : spectre conforme à la formule attendue.

- Spectre UV ($CHCl_3$) : $\lambda_{max}$ = 303 nm $\varepsilon$ = 15700
  $\lambda_{max}$ = 325 nm (épaulement) $\varepsilon$ = 12830

- Analyse élémentaire :

calculé :C 65,50; H 6,94; 0 27,55
trouvé :C 65,33; H 6,91; 0 27,78

EXEMPLE 3 :

3-allyl-4,5-diméthoxy-benzalmalonate de di-(2-éthylhexyle)

Préparation d'un composé de formule générale (I) dans laquelle $R_1$ représente le radical $-CH_2-CH=CH_2$, $R_2$ et $R_3$ représentent le radical $-OCH_3$ et $R_5$ et $R_6$ représentent le radical $-CH_2CH(C_2H_5)C_4H_9$.

On chauffe au reflux avec un Dean Stark un mélange de 3-allyl-4,5-diméthoxy-benzaldéhyde (10,3 g, 0,05 mole), de malonate de di-(2-éthylhexyle) (16,4 g, 0,05 mole), de toluène (20 ml), d'acide acétique (0,41 ml) et de pipéridine (0,77 ml). Après refroidissement, lavage à l'eau de la phase toluénique, séchage et évaporation du solvant, on obtient une huile orangée laquelle est purifiée par chromatographie sur colonne de silice 60 (éluant : heptane/acétate d'éthyle 90:10) pour donner le 3-allyl-4,5-diméthoxy-benzalmalonate de di-(2-éthylhexyle) (huile incolore, 15 g, rendement = 64%) possédant les caractéristiques suivantes :

- Spectre de RMN 1H ($CDCl_3$) : spectre conforme à la formule attendue.

- Spectre UV ($CHCl_3$) : $\lambda_{max}$ = 303 nm $\varepsilon$ = 15550
  $\lambda_{max}$ = 320 nm (épaulement) $\varepsilon$ = 13430

- Analyse élémentaire :

calculé : C 72,06; H 9,36; 0 18,58
trouvé : C 72,09; H 9,44; 0 18,69

EXEMPLE 4 :

3-allyl-4-butoxy-5-méthoxy-benzalmalonate de diéthyle

Préparation d'un composé de formule générale (I) dans laquelle $R_1$ représente le radical $-CH_2CH=CH_2$, $R_2$ représente le radical $-OC_4H_9$, $R_3$ représente le radical $-OCH_3$ et $R_5$ et $R_5$ représentent le radical $-C_2H_5$.

Premier stade :

On maintient pendant 3 heures à 40-45°C un mélange de 3-allyl-4-hydroxy-5-méthoxy-benzaldéhyde (10,25 g, 0,053 mole) de diméthylformamide (150 ml), de carbonate de potassium (8,29 g, 0,06 mole) et de 1-bromobutane (8,22 g, 0,06 mole). On plonge le mélange réactionnel dans de l'eau glacée et on extrait l'huile formée par du dichlorométhane. Après lavage à l'eau, séchage et évaporation du solvant, on obtient une huile brune laquelle est passée sur lit de silice 60 pour donner une huile jaune pâle de 3-allyl-4-butoxys-5-méthoxy-benzaldéhyde (13 g, rendement = 91%).

Deuxième stade :

On chauffe au reflux pendant 7 heures avec un Dean Stark un mélange du dérivé précédent (10,2 g, 0,041 mole), de malonate de diéthyle (7 g, 0,041 mole), de toluène (12 ml), d'acide acétique (0,26 ml) et de pipéridine (0,49 ml). De la même manière qu'à l'exemple 3, on obtient le 3-allyl-4-butoxy-5-méthoxy-benzalmalonate de diéthyle (huile incolore, 10 g, rendement = 67%) possédant les caractéristiques suivantes :
- Spectre RMN 1H ($CDCl_3$) : spectre conforme à la structure attendue.

- Spectre UV ($CH_2Cl_2$) : $\lambda_{max}$ = 305 nm $\varepsilon$ = 15500
$\lambda_{max}$ = 325 nm (épaulement) $\varepsilon$ = 13530

- Analyse élémentaire :
calculé : C 67,67; H 7,74; 0 24,58
trouvé : C 67,87; H 7,83; 0 24,44.

EXEMPLES D'APPLICATION

Exemple A - Huile antisolaire

On mélange les produits suivants en chauffant éventuellement à 40-45°C pour homogénéiser :

| | | |
|---|---|---|
| - Beurre de cacao | | 2,5 g |
| - Composé de l'exemple 2 | | 1,5 g |
| - Butylhydroxyanisole | | 0,05 g |
| - Parfum | qs | |
| - Huile végétale | qsp | 100 g |

Exemple B - Huile antisolaire

| | | |
|---|---|---|
| - Lanoline | | 2,5 g |
| - Composé de l'exemple 1 | | 3 g |
| - Butylhydroxyanisole | | 0,05 g |
| - Parfum | qs | |
| - Triglycérides d'acides en $C_8$-$C_{12}$ | qsp | 100 g |

Exemple C - Lotion oléo-alcoolique antisolaire

| | | |
|---|---|---|
| - Lanoline | | 2,5 g |
| - Triglycérides d'acides gras en $C_8$-$C_{12}$ | | 40 g |
| - Parfum | qs | |
| - Composé de l'exemple 1 | | 2 g |
| - Butylhydroxytoluène | | 0,05 g |
| - Alcool à 96° | qsp | 100 g |

## Revendications

1. Dérivé insaturé de benzalmalonate de formule :

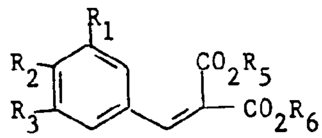

(I)

dans laquelle :

$R_1$ et $R_2$ représentent un atome d'hydrogène, un radical hydroxyle, un radical triméthylsiloxy, un radical alkyle en $C_1$-$C_6$ à chaîne droite ou ramifiée, un radical alcoxy en $C_1$-$C_6$ à chaîne droite ou ramifiée ou un radical $-(CH_2)_p-C(R_4)=CH_2$ dans lequel p représente un nombre entier compris entre 1 et 10 et de préférence entre 1 et 4 et $R_4$ représente un atome d'hydrogène ou un radical alkyle en $C_1$-$C_4$ à chaîne droite ou ramifiée, l'un des deux radicaux $R_1$ ou $R_2$ représentant un reste $-(CH_2)_p-C(R_4)=CH_2$,

$R_3$ représente un atome d'hydrogène, un radical alkyle en $C_1$-$C_6$ à chaîne droite ou ramifiée ou un radical alcoxy en $C_1$-$C_6$ à chaîne droite ou ramifiée,

$R_5$ et $R_6$ identiques ou différents représentent un radical alkyle en $C_1$-$C_8$ à chaîne droite ou ramifiée.

2. Composé selon la revendication 1, caractérisé par le fait qu'il est choisi parmi le 3-allyl-4-hydroxy-benzalmalonate de diéthyle, le 3-allyl-4-méthoxy-benzalmalonate de diéthyle, le 3-méthallyl-4-méthoxy-benzalmalonate de diéthyle, le 4-allyl-benzalmalonate de diéthyle, le 4-méthallyl-benzalmalonate de diéthyle, le 3-allyl-4,5-diméthoxy-benzalmalonate de diéthyle, le 3-allyl-4-butoxy-5-méthoxy-benzalmalonate de diéthyle, le 3-allyl-4,5-diméthoxy-benzalmalonate de di-(2-éthylhexyle).

3. Composition cosmétique, caractérisée par le fait qu'elle comprend une quantité efficace d'au moins un composé de formule (I) selon la revendication 1, dans un support cosmétiquement acceptable contenant au moins une phase grasse.

4. Composition cosmétique selon la revendication 3, caractérisée par le fait qu'elle comprend à titre de composé (I), au moins l'un des composés choisi parmi le 3-allyl-4-hydroxy-benzalmalonate de diéthyle, le 3-allyl-4-méthoxy-benzalmalonate de diéthyle, le 3-méthallyl-4-méthoxy-benzalmalonate de diéthyle, le 4-allyl-benzalmalonate de diéthyle, le 4-méthallyl-benzalmalonate de diéthyle, le 3-allyl-4,5-diméthoxy-benzalmalonate de diéthyle, le 3-allyl-4-butoxy-5-méthoxy-benzalmalonate de diéthyle, le 3-allyl-4,5-diméthoxy-benzalmalonate de di-(2-éthylhexyle).

5. Composition cosmétique selon la revendication 3 ou 4, caractérisée par le fait qu'elle se présente sous forme de lotion huileuse ou oléoalcoolique, d'émulsion, gel oléoalcoolique, alcoolique ou hydroalcoolique, bâtonnet solide ou aérosol.

6. Composition cosmétique selon la revendication 5, caractérisée par le fait qu'elle contient en outre des adjuvants cosmétiques choisis parmi les épaississants, adoucissants, humectants, tensio-actifs, conservateurs, anti-mousses, parfums, huiles, cires, lanoline, monoalcools et polyols inférieurs, propulseurs, colorants et pigments.

7. Composition cosmétique selon l'une quelconque des revendications 3 à 6, caractérisée par le fait qu'elle constitue une composition protectrice de l'épiderme humain et contient 0,25 à 3% en poids de composé de formule (I).

8. Composition cosmétique selon l'une quelconque des revendications 3 à 6, se présentant sous forme de composition anti-solaire, caractérisée par le fait qu'elle contient 0,5 à 15% en poids de composé de formule (I).

9. Composition cosmétique anti-solaire selon la revendication 8, caractérisée par le fait qu'elle contient en outre un agent filtrant les rayons UV-B et/ou UV-A.

10. Composition cosmétique selon la revendication 3 ou 4, destinée à être appliquée sur les cheveux, caractérisée par le fait qu'elle se présente sous forme de shampooing, lotion, gel ou émulsion à rincer, lotion ou gel coiffants ou traitants, lotion ou gel pour le brushing ou la mise en plis, laque pour cheveux, composition de permanente, de décoloration ou de coloration et comprend 0,25 à 3% en poids de composé de formule (I).

11. Composition cosmétique selon la revendication 3 ou 4, se présentant sous forme d'une composition cosmétique colorée ou non, caractérisée par le fait qu'elle est constituée par une composition capillaire, un produit de maquillage ou une composition pour les soins ou le traitement de la peau, comprenant 0,25 à 3% en poids de composé de formule (I).

12. Procédé de protection de la peau et des cheveux naturels ou sensibilisés contre le rayonnement ultraviolet, caractérisé par le fait qu'il consiste à appliquer sur la peau ou les cheveux une quantité efficace d'une

composition cosmétique contenant au moins un dérivé insaturé de benzalmalonate de formule (I) selon la revendication 1 ou 2.

13. Procédé de protection d'une composition cosmétique contre les rayons ultraviolets, caractérisé par le fait qu'il consiste à incorporer à cette composition une quantité efficace d'au moins un composé de formule (I) selon la revendication 1 ou 2.

## Patentansprüche

1. Ungesättigtes Derivat von Benzalmalonat mit der Formel (I)

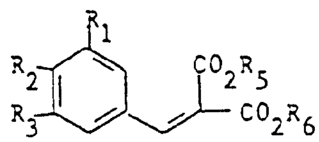

(I)

worin

$R_1$ und $R_2$ ein Wasserstoffatom, ein Hydroxylradikal, ein Trimethylsiloxyradikal, ein $C_{1-6}$-Alkylradikal mit gerader oder verzweigter Kette, ein $C_{1-6}$-Alkoxyradikal mit gerader oder verzweigter Kette oder ein $-(CH_2)_p-C(R_4)=CH_2$-Radikal bedeuten, worin p eine ganze Zahl zwischen 1 und 10 und vorzugsweise zwischen 1 und 4 ist, und worin $R_4$ ein Wasserstoffatom oder ein $C_{1-4}$-Alkylradikal mit einer geraden oder verzweigten Kette darstellt, wobei eines der beiden Radikale $R_1$ oder $R_2$ einen Rest $-(CH_2)_p-C(R_4)=CH_2$ darstellen,

worin $R_3$ ein Wasserstoffatom, ein $C_{1-6}$-Alkylradikal mit gerader oder verzweigter Kette oder ein $C_{1-6}$-Alkoxyradikal mit gerader oder verzweigter Kette bedeutet;

worin $R_5$ und $R_6$, identisch oder verschieden, ein $C_{1-8}$-Alkylradikal mit gerader oder verzweigter Kette bedeuten.

2. Verbindung nach Anspruch 1, dadurch **gekennzeichnet**, dass sie ausgewählt ist aus Diethyl-3-allyl-4-hydroxy-benzalmalonat, Diethyl-3-allyl-4-methoxy-benzalmalonat, Diethyl-3-methallyl-4-methoxy-benzalmalonat, Diethyl-4-allyl-benzalmalonat, Diethyl-4-methallyl-benzalmalonat, Diethyl-3-allyl-4,5-dimethoxy-benzalmalonat, Diethyl-3-allyl-4-butoxy-5-methoxy-benzalmalonat und Di-(2-ethylhexyl)-3-allyl-4,5-dimethoxy-benzalmalonat.

3. Kosmetikzusammensetzung, dadurch **gekennzeichnet**, dass sie eine wirksame Menge von wenigstens einer Verbindung der Formel (I) nach Anspruch 1 in einem kosmetisch akzeptablen Träger umfasst, der zumindest eine Fettphase aufweist.

4. Kosmetikzusammensetzung nach Anspruch 3, dadurch **gekennzeichnet**, dass sie als Verbindung (I) zumindest eine der Verbindungen enthält, ausgewählt aus Diethyl-3-allyl-4-hydroxy-benzalmalonat, Diethyl-3-allyl-4-methoxy-benzalmalonat, Diethyl-3-methallyl-4-methoxy-benzalmalonat, Diethyl-4-allyl-benzalmalonat, Diethyl-4-methallyl-benzalmalonat, Diethyl-3-allyl-4,5-dimethoxy-benzalmalonat, Diethyl-3-allyl-4-butoxy-5-methoxy-benzalmalonat und Di-(2-ethylhexyl)-3-allyl-4,5-dimethoxy-benzalmalonat.

5. Kosmetikzusammensetzung nach Anspruch 3 oder 4, dadurch **gekennzeichnet**, dass sie in der Form einer ölhaltigen oder ölalkoholischen Lotion, einer Emulsion, eines ölalkoholischen, alkoholischen oder hydroalkoholischen Gels, eines festen Stäbchens oder in Form eines Aerosols vorliegt.

6. Kosmetikzusammensetzung nach Anspruch 5, dadurch **gekennzeichnet**, dass sie zusätzlich kosmetische Hilfsmittel enthält, ausgewählt aus Verdickungsmitteln, Weichmachern, Anfeuchtemitteln, oberflächenaktiven Mitteln, Konservierungsmitteln, Antischaummitteln, Parfüms, Öle, Wachse, Lanolin, niederen Monoalkoholen und Polyolen, Treibmitteln, Färbemitteln und Pigmenten.

7. Kosmetikzusammensetzung nach einem der Ansprüche 3 bis 6, dadurch **gekennzeichnet**, dass sie eine Zusammensetzung darstellt, die die menschliche Haut schützt, und dass sie 0,25 bis 3 Gew.% der Verbindung der Formel (I) enthält.

8. Kosmetikzusammensetzung nach einem der Ansprüche 3 bis 6, die sich als Sonnenschutzzusammensetzung darstellt, dadurch **gekennzeichnet**, dass sie 0,5 bis 15 Gew.% der Verbindung der Formel (I) enthält.

9. Sonnenschutz-Kosmetikzusammensetzung nach Anspruch 8, dadurch **gekennzeichnet**, dass sie zusätzlich ein Mittel enthält, das die UV-B- und/oder UV-A-Strahlen filtert.

10. Kosmetikzusammensetzung nach Anspruch 3 oder 4 zur Anwendung bei den Haaren, dadurch **gekennzeichnet**, dass sie in der Form eines Shampoos, einer Lotion, eines Spülgels oder einer Spülemulsion, einer Frisier- oder Behandlungs-Lotion oder -Gels, einer Lotion oder eines Gels zum Kämmen oder zum Legen

von Wasserwellen, eines Haarlackes, einer Dauerwellenzusammensetzung, Entfärbungszusammensetzung oder Färbungszusammensetzung vorliegt und dass sie 0,25 bis 3 Gew.% der verbindung der Formel (I) enthält.

11. Kosmetikzusammensetzung nach Anspruch 3 oder 4, die in der Form einer Kosmetikzusammensetzung vorliegt, die gefärbt oder nicht gefärbt ist, dadurch **gekennzeichnet**, dass sie durch eine Haarzusammensetzung, ein Schminkprodukt oder eine Zusammensetzung für die Pflege oder die Behandlung der Haut gebildet ist, wobei sie 0,25 bis 3 Gew.% der verbindung der Formel (I) enthält.

12. verfahren zum Schutz der Haut und der natürlichen oder der empfindlichen Haare gegen ultraviolette Bestrahlung, **gekennzeichnet** durch Auftragen auf die Haut oder die Haare einer wirksamen Menge einer Kosmetikzusammensetzung, die zumindest ein ungesättigtes Derivat von Benzalmalonat der Formel (I) nach Anspruch 1 oder 2 enthält.

13. verfahren zum Schutz einer Kosmetikzusammensetzung gegen ultraviolette Strahlen, **gekennzeichnet** durch Eingeben einer wirksamen Menge von zumindest einer Verbindung der Formel (I) nach Anspruch 1 oder 2 in diese Zusammensetzung.

## Claims

1. Unsaturated benzalmalonate derivative of formula:

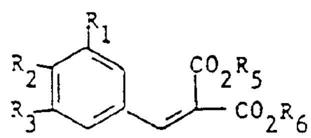

(I)

in which:

$R_1$ and $R_2$ denote a hydrogen atom, a hydroxyl radical, a trimethylsiloxy radical, a $C_1$-$C_6$ straight or branched chain alkyl radical, a $C_1$-$C_6$ straight or branched chain alkoxy radical or a radical $-(CH_2)p-C(R_4)=CH_2$, in which p denotes an integer between 1 and 10 and preferably between 1 and 4 and $R_4$ denotes a hydrogen atom or a $C_1$-$C_4$ straight or branched chain alkyl radical, one of the two radicals $R_1$ or $R_2$ denoting a residue $-(CH_2)p-C(R_4)=CH_2$,

$R_3$ denotes a hydrogen atom, a $C_1$-$C_6$ straight or branched chain alkyl radical or a $C_1$-$C_6$ straight or branched chain alkoxy radical,

$R_5$ and $R_5$, which are identical or different, denote a $C_1$-$C_8$ straight or branched chain alkyl radical.

2. Compound according to Claim 1, characterised in that it is chosen from diethyl 3-allyl-4-hydroxybenzalmalonate, diethyl 3-allyl-4-methoxybenzalmalonate, diethyl 3-methallyl-4-methoxybenzalmalonate, diethyl 4-allylbenzalmalonate, diethyl 4-methallylbenzalmalonate, diethyl 3-allyl-4,5-dimethoxybenzalmalonate, diethyl 3-allyl-4-butoxy-5-methoxybenzalmalonate and di-(2-ethylhexyl) 3-allyl-4,5-dimethoxybenzalmalonate.

3. Cosmetic composition characterised in that it comprises an effective quantity of at least one compound of formula (I) according to Claim 1, in a cosmetically acceptable substrate containing at least one fatty phase.

4. Cosmetic composition according to Claim 3, characterised in that it comprises, as compound (I), at least one of the compounds chosen from diethyl 3-allyl-4-hydroxybenzalmalonate,diethyl 3-allyl-4-methoxybenzalmalonate, diethyl 3-methallyl-4-methoxybenzalmalonate, diethyl 4-allylbenzalmalonate, diethyl 4-methallylbenzalmalonate, diethyl 3-allyl-4,5-dimethoxybenzalmalonate, diethyl 3-allyl-4-butoxy-5-methoxybenzalmalonate and di-(2-ethylhexyl) 3-ally-4,5-dimethoxybenzalmalonate.

5. Cosmetic composition according to Claim 3 or 4, characterised in that it is in the form of an oily or oleoalcoholic lotion, emulsion, oleoalcoholic, alcoholic or hydroalcoholic gel, a solid stick or an aerosol.

6. Cosmetic composition according to Claim 5, characterised in that it additionally contains cosmetic adjuvants chosen from thickeners, softeners, humectants, surfactants, preserving agents, antifoams, perfumes, oils, waxes, lanolin, lower monoalcohols and polyols, propellants, colorants and pigments.

7. Cosmetic composition according to any one of Claims 3 to 6, characterised in that it forms a composition for protecting human skin and contains 0.25 to 3% by weight of compound of formula (I).

8. Cosmetic composition according to any one of Claims 3 to 6, which is in the form of a sunscreen composition, characterised in that it contains 0.5 to 15% by weight of compound of formula (I).

9. Cosmetic sunscreen composition according to Claim 8, characterised in that it additionally contains an agent filtering the UV-B and/or UV-A rays.

10. Cosmetic composition according to Claim 3 or 4, intended to be applied to hair, characterised in that it is in the form of a shampoo, lotion, gel or emulsion for rinsing, setting or treating lotion or gel, a lotion or gel

for blow drying or setting, hair lacquer, a composition for permanent-waving, bleaching or dyeing and comprises 0.25 to 3% by weight of compound of formula (I).

11. Cosmetic composition according to Claim 3 or 4, which is in the form of a coloured or uncoloured cosmetic composition, characterised in that it consists of a haircare composition, a makeup product or a composition for skin care or treatment, comprising 0.25 to 3% by weight of compound of formula (I).

12. Process for protecting skin and natural or sensitised hair against ultraviolet radiation, characterised in that it consists in applying to the skin or hair an effective quantity of a cosmetic composition containing at least one unsaturated benzalmalonate derivative of formula (I) according to Claim 1 or 2.

13. Process for protecting a cosmetic composition against ultraviolet rays, characterised in that it consists incorporating in this composition an effective quantity of at least one compound of formula (I) according to Claim 1 or 2.